# EUROPEAN PATENT SPECIFICATION

(11) **EP 3 903 055 B1**
(45) Date of publication and mention of the grant of the patent: **11.12.2024**
(21) Application number: 19839512.1
(22) Date of filing: 17.12.2019
(51) Int. Cl.: F25D 29/00, G01N 33/02, G01N 21/65

(54) **FOOD MONITORING ASSEMBLY AND METHOD**
ANORDNUNG UND VERFAHREN ZUR LEBENSMITTELÜBERWACHUNG
ENSEMBLE ET PROCÉDÉ DE CONTRÔLE DE LA QUALITÉ DES ALIMENTS

(30) Priority: 24.12.2018 IN 201811048838
(43) Date of publication of application: 03.11.2021
(73) Proprietor: Carrier Corporation, Palm Beach Gardens, FL 33418 (US)
(72) Inventor: MISHRA, Preeti K., Telagana, Hyderabad 500081 (IN); ANUMACHPALLI, Chakravarty, Telagana, Hyderabad 500081 (IN); KANKANE, Rahul, Telagana, Hyderabad 500081 (IN)
(74) Representative: Dehns
(86) International application number: PCT/US2019/066838
(87) International publication number: WO 2020/139625

(56) References cited:
- CN-A- 104 897 584
- CN-U- 205 784 217
- US-A1- 2016 138 857
- US-A1- 2016 321 951
- US-A1- 2017 082 540
- US-A1- 2018 322 436

## Description

This application relates to a food monitoring assemblies and methods, particularly related to monitoring food condition and extending shelf life.

Food waste is a significant problem with approximately thirty percent of food in the United States spoiling between production and consumption. Spoiled food may also need to be separated from non-spoiled food to prevent further spoilage. Therefore, there is a need to reduce food waste and identify food once it has spoiled.

US 2016/138857 A1 discloses a method of determining food freshness in a refrigerator using image data obtained with a camera.

CN 205 784 217 U discloses a food storage unit where food spoilage is determined with a color sensor and a gas sensor.

US 2016/321951 A1 discloses a food apparatus for detection of food type, with a battery charged by a solar cell.

According a first aspect the invention, a food monitoring assembly according to claim 1 is provided.

In a further embodiment of any of the above, at least one solar cell may be in electrical communication with a battery for powering the assembly.

In a further embodiment of any of the above, the color sensor may be configured to measure a wave length of light to determine the color of the food product.

In a further embodiment of any of the above, the controller may include a microprocessor and memory for storing food product data.

In a further embodiment of any of the above, the communicator may include a wireless connection for communicating with the remote location.

In a further embodiment, a refrigerated cooler may be in combination with the food monitor assembly, wherein the food monitor assembly may be located adjacent to the refrigerated cooler.

In a further embodiment, a food transport trailer may be combined with the food monitor assembly, wherein the food monitor assembly may be located within the food transport trailer.

According to another aspect of the invention a method of monitoring a condition of a food product according to claim 10 is provided.

In a further embodiment of any of the above, the step of signaling to the user may include illuminating a light.

In a further embodiment of any of the above, the user may be signaled if the gas emissions level is outside of a predetermined gas emissions range.

In a further embodiment of any of the above, the current color of the food product may be compared with a previously measured color of the food product to determine a color change of the food product.

In a further embodiment of any of the above, a refrigerated system may be adjusted to reduce food spoilage if the color change of the food product exceeds a predetermined level.

In a further embodiment of any of the above, food product data for the food product may be accessed after the type of food product has been identified.

Figure 1 illustrates an example refrigerated cooler including a food monitor.
Figure 2 illustrates an enlarged view of the food monitor in the refrigerated cooler adjacent a food product.
Figure 3 illustrates an example refrigerated container having multiple compartments each with at least one food monitor.
Figure 4 illustrates an example method of monitoring a condition of the food product.

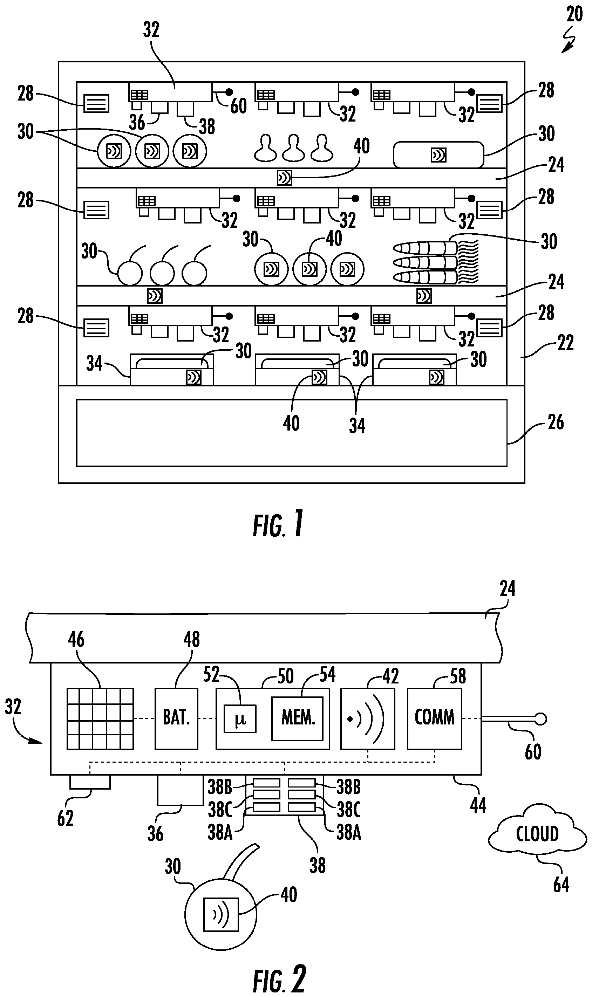

Figure 1 illustrates a refrigerated cooler 20 for storing a plurality of food products 30. In the illustrated example, the plurality of food products 30 can include produce, seafood, and/or dairy products. The refrigerated cooler 20 includes a cooler housing 22 that supports shelves 24 for displaying the food products 30 to customers, such as in a supermarket. The refrigerated cooler 20 also includes a refrigeration system 26 integrated into the cooler housing 22 to provide conditioned air to each of the plurality of shelves 24 through vents 28 in fluid communication with the refrigeration system 26 to extend a shelf life of the food products 30.

Food monitors 32 are located on the cooler housing 22 adjacent the plurality of food products 30 with at least one food monitor 32 located adjacent one of the vents 28. The food monitors 32 can identify the food product 30 in the vicinity of the food monitor 32 and then monitor a condition of the food product 30.

To identify the food product 30 in the vicinity of the food monitor 32, each of the food monitors 32 includes a RFID tag reader 42 (Figure 2) for reading a RFID tag 40 associated with each individual food product 30 or type of food product 30. In one example, the RFID tag 40 is a sticker and is located directly on the individual food product 30. In another example, the RFID tag 40 is located on a container 34 storing the food product 30. Although the illustrated example only shows three food monitors 32 associated with each shelf 24, the number of food monitors 32 associated with the refrigerated cooler 20 can vary depending on the number of different food products 30 stored in the refrigerated cooler 20 or the quantity of food products 30 stored in the refrigerated cooler 20.

Figure 2 illustrates an enlarged view of the food monitor 32 located on an underside of the shelf 24 and adjacent the food product 30. In the illustrated example, the food product 30 includes the RFID tag 40 attached directly to the food product through the use of a sticker. The food monitor 32 is attached to an underside of the shelf 24. However, the food monitor 32 could be located on another portion of the cooler housing 22 of the refrigerated cooler 20 that provides the food monitor 32 an unobstructed view of the food product 30.

In the illustrated non-limiting example, the food monitor 32 includes a monitor housing 44 that is attached directed to the shelf 24. The monitor housing 44 includes a controller 50 having a microprocessor 52 and memory 54 for managing operation of the food monitor 32. The food monitor 32 can be powered in at least one of two ways. In one example, the food monitor 32 receives power directly from the refrigerated cooler 20. In another example, the food monitor 32 utilizes at least one solar cell 46 to harvest energy from the environment surrounding the refrigerated cooler 20. The energy generated from the at least one solar cell 46 can directly power the food monitor 32 or charge a battery 48 in electrical communication with the at least one solar cell 46. The battery 48 allows the food monitor 32 to continue operating when the at least one solar cell 46 is not generating enough power such as during night time operations.

The food monitor 32 also includes the RFID tag reader 42 in electrical communication with the controller 50 for identifying the food product 30. The controller 50 can access food product data stored in the memory 54 to determine an appropriate color range and pesticide level for the food product 30. The controller 50 can access data stored remotely, such as cloud data 64 stored in the cloud, through a communicator 58 having an antenna 60 through a Bluetooth or other wireless connection. Alternatively, the communicator 58 can be hard wired into a network for accessing the food product data stored remotely.

Once the food monitor 32 has identified the adjacent food product 30 and accessed the associated food product data, the food monitor 32 can measure a current color of the adjacent food product 30 with a color sensor 36. The color sensor 36 is configured to measure a wave length of light reflected off of the food product to determine the current color of the adjacent food product 30. The controller 50 can compare the current color of the food product 30 with a previously measured color of the food product 30 stored in the memory 54 and with an acceptable food product color range stored in the memory 54 to determine a condition of the food product 30. Alternatively, the controller 50 can compare the current color of the food product 30 with a previously measured color of the food product stored in the cloud data 64 and with an acceptable food product color range stored in the cloud.

The food monitor 32 also includes a biosensor 38 for measuring gas emissions from the food products 30 and/or determining a pesticide level associated with the food product 30. The biosensor 38 includes at least one chemical sensor for measuring food product gas emissions and pesticide levels. The at least one chemical sensor includes a reactive bio-component element 38A, a sensor element 38B, and an interface element 38C disposed there between. The bio-component element 38A includes a bio-agent, such as bioactive species or biomimetic species, selected to interact specifically with a particular analyte to be sensed. The bio-agent, typically through a biochemical process, acts to bind or convert the analyte into a measurable component. The bio-component element 38A used in the illustrated example includes biological species such as enzymes, antigens, antibodies, receptors, tissues, whole cells, cell organelles, bacteria, and nucleic acids.

The sensor element 38B includes a physical component operative to generate a measurable output, usually an electrical or optical signal, indicative of the presence of the analyte and, in certain instances, the actual amount of the analyte that is received by the controller 50. The sensor element 38B includes at least one of an electrochemical device, an optical device, an acoustical device, or a calorimetric device.

The interface element 38C includes a membrane or coating that separates the sensor element 38B from the bio-component element 38A and serves as a link between the elements. In the illustrated example, the interface element 38C includes at least one of a polymer membrane, an electropolymerized coating, or a self-assembling monomers.

The biosensor 38 can monitor gas emissions from the food products 30 in the vicinity. Alternatively, the food monitor 32 can be located adjacent one of the vents 28 to determine if one of the food products 30 somewhere associated with the refrigerated cooler 20 is spoiling. The food monitor 32 sends a notification to a user regarding a possible food spoilage issue and directs the refrigeration system 26 to operate at a lower temperature to decrease the rate of spoilage or prevent the spoilage of the food products 30 from spreading. Alternatively, the food monitor 32 can illuminate a light 62 to indicate an undesirable condition of the food product 30. For example, the light 62 can illuminate red if risk of food spoilage is present or illuminate green if no risk is identified for spoilage.

It is also possible that the food products 30 may not be fit for consumption due to unacceptable levels of pesticides present with the food products 30. If the food monitor 32 determines that the food products 30 include an unacceptable level of pesticides, the food monitor 32 sends a notification to the user, such as a seller of the food products 30, of the possibility of pesticide contamination. The notification can be sent through the communicator 58 and the antenna 60 to the user or the food monitor 32 can illuminate the light 62 to indicate that the particular food product 30 is not fit for consumption. For example, the light 62 can illuminate red to indicate that the food product 30 is not acceptable for consumption or the light 62 can illuminate green to indicate that the food product 30 is acceptable for consumption.

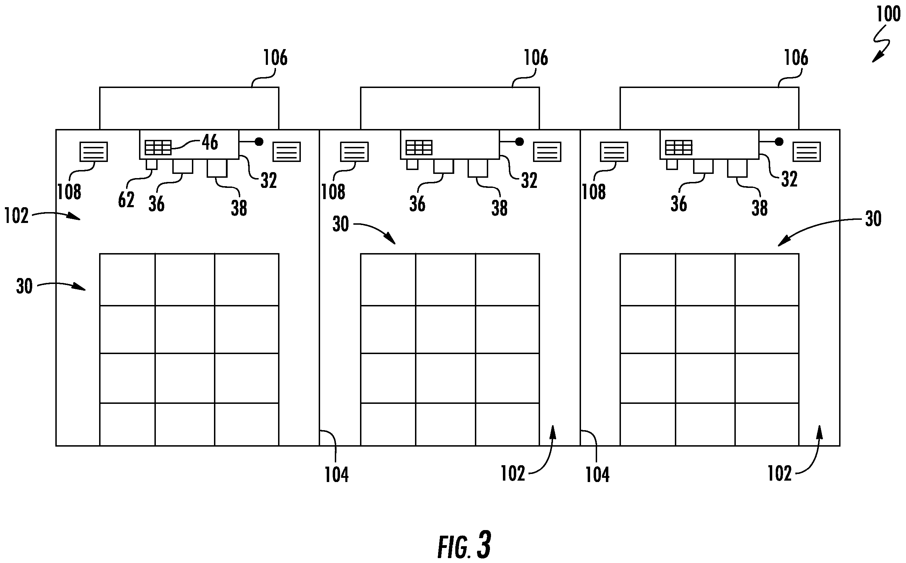

Figure 3 illustrates an example refrigerated container 100 including multiple individual compartments 102 separated by divider walls 104 to allow the food products 30 to be stored at different temperatures. Each of the individual compartments 102 include at least one food monitor 32 that operates in a similar manner as the food monitors 32 described above with respect to the refrigerated cooler 20.

In particular, the food monitor 32 can use the color sensor 36 and biosensor 38 to monitor a condition of the food products 30 in each of the individual compartments 102 and communicate the condition of the food products 30 to the user as described above. However, because the individual compartments 102 rely on a greater degree of air circulation than the refrigerated cooler 20 above, positioning one of the food monitors 32 adjacent a vent 108 may be more effective in capturing the circulation of gas emissions from spoiling food. Additionally, the at least one solar cell 46 on the food monitor 32 may be less effective for the refrigerated container 100 because the individual compartments 102 for the refrigerated container 100 have less light exposure than the refrigerated cooler 20 described above.

The food monitors 32 in the refrigerated container 100 can work in conjunction with refrigeration systems 106 to adjust a temperature for the individual compartments 102. By adjusting the temperature for the individual compartments 102 separately and based on information obtained from the food monitors 32 regarding the condition of the food products 30 located within the individual compartments 102, the food products 30 stay fresh for longer periods of time, which reduces waste of the food products 30.

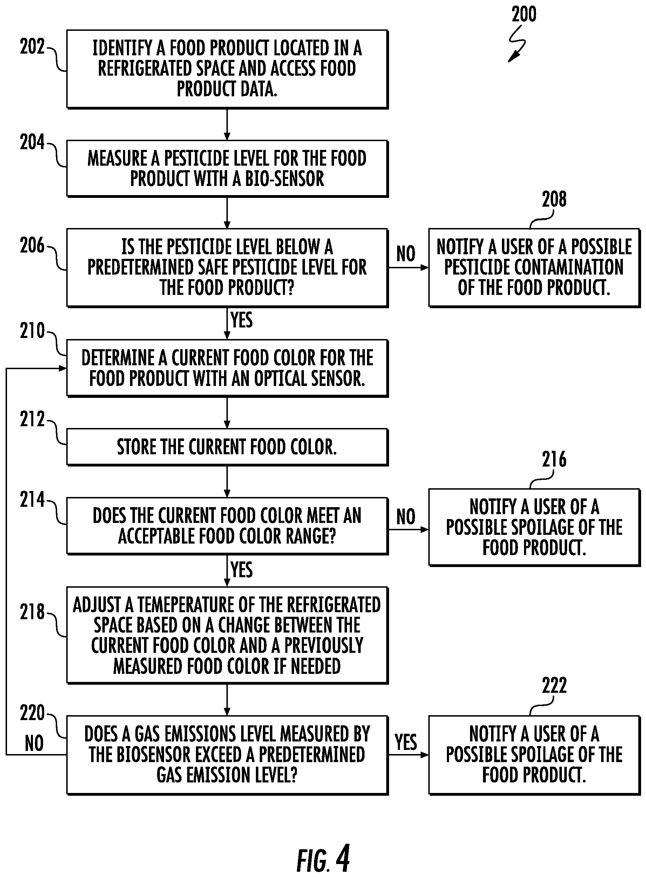

Figure 4 illustrates a method of operating the food monitors 32 with either the refrigerated cooler 20 or the refrigerated container 100 to extend the freshness of the food products 30 and reduce waste. The food monitor 32 initially identifies the food product 30 located in the vicinity of the food monitor 32 through the use of the RFID tag reader 42. Block 202. Once the RFID tag reader 42 has identified the specific food product 30 associated with the RFID tag 40, the food monitor 32 is able to access food product data for the specific food product 30. The food product data can either be stored in the memory 54 in the food monitor 32 or accessed remotely by the food monitor 32, such as by accessing the cloud data 64.

Once the food monitor has identified the specific type of the food product 30 and accessed the associated food product data, the food monitor 32 measures a pesticide level associated with the food product 30 with the biosensor 38. Block 204. The food monitor 32 then determines if the measured pesticide level for the particular food product 30 is below a predetermined safe pesticide level for the food product 30. Block 206. If the measured pesticide level for the food product 30 exceeds the predetermined safe pesticide level, the food monitor 32 notifies the user of the possibility of pesticide contamination of the food product 30. Block 208.

If the pesticide level is below the predetermined safe pesticide level, the food monitor 32 will then determine a current food color for the food product 30 with the color sensor 36. Block 210. The current food color is then stored in at least one of the memory 54 or the cloud data 64 for analyzing changes in the food products 30. Block 212.

The food monitor 32 then determines if the current food color is within an acceptable food color range for the particular food product 30. Block 214. If the current food color is not within the acceptable food color range, the food monitor 32 notifies the user of the possibility of spoiled food products 30. Block 216.

The food monitor 32 then determines a change in color for the food product by comparing the current food color with a previously measured food color stored in the cloud data 64 or the memory 54. The food monitor 32 can communicate with the refrigeration system 26, 106 to adjust the temperature in the vicinity of the food products 30 to extend the life of the food product 30 if the change in color between the current food color and the previously measured food color is larger than a predetermined valve. Block 218.

The food monitor 32 also measures for the presence of a gas emission indicative of the food product 30 spoiling in the vicinity of the food monitor 32 and if the gas emission level exceeds a predetermined level. Block 220. If the measured gas emission level exceeds the predetermined level, the food monitor 32 notifies the user of the possibility of food product 30 spoilage. Block 222. The food monitor 32 can communicate with the refrigeration system 26, 106 to adjust the temperature in the vicinity of the food products 30 to extend the life of the food product 30 if the gas emission level exceeds the predetermined level.

If the biosensor 38 does not identify gas emissions beyond the acceptable emissions level, the method 200 returns to block 210 and will determine a new current food color for the food product 30 with the color sensor 36 and will repeat the above described comparisons to monitor for the food products 30 spoiling.

The preceding description is exemplary rather than limiting in nature. Variations and modifications to the disclosed examples may become apparent to those skilled in the art. The scope of legal protection given to this disclosure can only be determined by studying the following claims.

## Claims

1. A food monitoring assembly (32) comprising:
a color sensor (36) configured to measure a color of a food product (30);
a RFID tag reader (42) configured to identify a RFID tag (40) on the food product (30);
a controller (50) in electrical communication with the color sensor (36) and the RFID tag reader (42) and configured to identify a type of the food product (30) based on data from the RFID tag reader (42); and
a communicator (58) in electrical communication with the controller (50) for communication with a remote location,
**characterised in that** the controller (50) is configured to:
determine an acceptable color range for the food product (30) based on the identification of the type of the food product (30); and
determine if a current color of the food product (30) measured by the color sensor is within the acceptable color range.

2. The assembly of claim 1, further comprising at least one biosensor in electrical communication with the controller.

3. The assembly of claim 2, wherein the at least one biosensor is configured to measure gas emissions emitted from the food product.

4. The assembly of claim 2, wherein the at least one biosensor includes at least one chemical sensor having a reactive bio-component element, a sensor element, and an interface element disposed there between.

5. The assembly of claim 1, further comprising at least one solar cell (46) in electrical communication with a battery (48) for powering the assembly (32).

6. The assembly of any of claims 1-5, wherein the food monitor assembly (32) is located adjacent to the refrigerated cooler (20).

7. The assembly of claim 1, wherein the controller is further configured to adjust the temperature of a refrigeration system to reduce food spoilage if the color change of the food product exceeds a predetermined level.

8. The assembly of any of claims 1-5, wherein the food monitor assembly (32) is located within a food transport trailer.

9. The assembly of claim 1, wherein the controller includes a microprocessor and memory for storing food product data, wherein the food product data includes the acceptable color range for the food product.

10. A method of monitoring a condition of a food product (30) comprising the steps of:
identifying a type of the food product based on data from a RFID tag reader (42) reading a RFID tag on the food product
determining an acceptable range of colors for the food product (30) based on the identification of the type of the food product (30);
measuring a current color of the food product (30) with at least one color sensor (36);
determining if the current color is within the acceptable range of colors for the food product (30); and
signaling to a user if the current color is outside of the acceptable range of colors for the food product (30).

11. The method of claim 10, wherein the step of signaling to the user includes illuminating a light (62).

12. The method of claim 10, further comprising measuring a gas emissions level associated with the food product (30) using at least one biosensor.

13. The method of claim 12, further comprising signaling to the user if the gas emissions level is outside of a predetermined gas emissions range.

14. The method of claim 10, further comprising adjusting a temperature of a refrigerated system to reduce food spoilage if the color change of the product exceeds a predetermined level.

15. The method of claim 10, wherein the step of determining an acceptable range of colors for the food product (30) includes accessing stored food product data associated with the type of the food product (30).

## Patentansprüche

1. Lebensmittelüberwachungsanordnung (32), umfassend:
einen Farbsensor (36), der dazu konfiguriert ist, eine Farbe eines Lebensmittelprodukts (30) zu messen;
ein RFID-Etikettenlesegerät (42), das dazu konfiguriert ist, ein RFID-Etikett (40) auf dem Lebensmittelprodukt (30) zu identifizieren;
eine Steuerung (50) in elektrischer Kommunikation mit dem Farbsensor (36) und dem RFID-Etikettenlesegerät (42) und dazu konfiguriert, einen Typ des Lebensmittelprodukts (30) basierend auf Daten von dem RFID-Etikettenlesegerät (42) zu identifizieren; und
einen Kommunikator (58) in elektrischer Kommunikation mit der Steuerung (50) zur Kommunikation mit einem entfernten Standort, **dadurch gekennzeichnet, dass** die Steuerung (50) konfiguriert ist zum:
Bestimmen eines akzeptablen Farbbereichs für das Lebensmittelprodukt (30) basierend auf der Identifizierung des Typs des Lebensmittelprodukts (30); und
Bestimmen, ob eine aktuelle Farbe des von dem Farbsensor gemessenen Lebensmittelprodukts (30) innerhalb des akzeptablen Farbbereichs liegt.

2. Anordnung nach Anspruch 1, ferner umfassend mindestens einen Biosensor in elektrischer Kommunikation mit der Steuerung.

3. Anordnung nach Anspruch 2, wobei der mindestens eine Biosensor dazu konfiguriert ist, von dem Lebensmittelprodukt emittierte Gasemissionen zu messen.

4. Anordnung nach Anspruch 2, wobei der mindestens eine Biosensor mindestens einen chemischen Sensor mit einem reaktiven Biokomponentenelement, einem Sensorelement und einem dazwischen angeordneten Schnittstellenelement beinhaltet.

5. Anordnung nach Anspruch 1, ferner umfassend mindestens eine Solarzelle (46) in elektrischer Kommunikation mit einer Batterie (48) zum Antreiben der Anordnung (32).

6. Anordnung nach einem der Ansprüche 1-5, wobei die Lebensmittelüberwachungsanordnung (32) sich benachbart zu der gekühlten Kühlvorrichtung (20) befindet.

7. Anordnung nach Anspruch 1, wobei die Steuerung ferner dazu konfiguriert ist, die Temperatur eines Kühlsystems zu justieren, um einen Verderb von Lebensmitteln zu verringern, wenn die Farbveränderung des Lebensmittelprodukts ein vorbestimmtes Niveau überschreitet.

8. Anordnung nach einem der Ansprüche 1-5, wobei die Lebensmittelüberwachungsanordnung (32) sich innerhalb eines Lebensmitteltransportanhängers befindet.

9. Anordnung nach Anspruch 1, wobei die Steuerung einen Mikroprozessor und einen Speicher zum Speichern von Lebensmittelproduktdaten beinhaltet, wobei die Lebensmittelproduktdaten den akzeptablen Farbbereich für das Lebensmittelprodukt beinhalten.

10. Verfahren zum Überwachen eines Zustands eines Lebensmittelprodukts (30), umfassend die Schritte:
Identifizieren eines Typs des Lebensmittelprodukts basierend auf Daten von einem RFID-Etikettenlesegerät (42), das ein RFID-Etikett auf dem Lebensmittelprodukt liest
Bestimmen eines akzeptablen Bereichs von Farben für das Lebensmittelprodukt (30) basierend auf der Identifizierung des Typs des Lebensmittelprodukts (30);
Messen einer aktuellen Farbe des Lebensmittelprodukts (30) mit mindestens einem Farbsensor (36);
Bestimmen, ob die aktuelle Farbe innerhalb des akzeptablen Bereichs von Farben für das Lebensmittelprodukt (30) liegt; und
Signalisieren an einen Benutzer, wenn die aktuelle Farbe außerhalb des akzeptablen Bereichs von Farben für das Lebensmittelprodukt (30) liegt.

11. Verfahren nach Anspruch 10, wobei der Schritt des Signalisierens an den Benutzer ein Aufleuchtenlassen eines Lämpchens (62) beinhaltet.

12. Verfahren nach Anspruch 10, ferner umfassend ein Messen eines Gasemissionsniveaus, das mit dem Lebensmittelprodukt (30) assoziiert ist, unter Verwendung mindestens eines Biosensors.

13. Verfahren nach Anspruch 12, ferner umfassend ein Signalisieren an den Benutzer, wenn das Gasemissionsniveau außerhalb eines vorbestimmten Gasemissionsbereichs liegt.

14. Verfahren nach Anspruch 10, ferner umfassend ein Justieren einer Temperatur eines gekühlten Systems, um einen Verderb von Lebensmitteln zu verringern, wenn die Farbveränderung des Produkts ein vorbestimmtes Niveau überschreitet.

15. Verfahren nach Anspruch 10, wobei der Schritt des Bestimmens eines akzeptablen Bereichs von Farben für das Lebensmittelprodukt (30) ein Zugreifen auf gespeicherte Lebensmittelproduktdaten beinhaltet, die mit dem Typ des Lebensmittelprodukts (30) assoziiert sind.

## Revendications

1. Ensemble de contrôle de la qualité des aliments (32) comprenant :
un capteur de couleur (36) configuré pour mesurer la couleur d'un produit alimentaire (30) ;
un lecteur d'étiquette RFID (42) configuré pour identifier une étiquette RFID (40) sur le produit alimentaire (30) ;
un dispositif de commande (50) en communication électrique avec le capteur de couleur (36) et le lecteur d'étiquette RFID (42) et configuré pour identifier un type du produit alimentaire (30) sur la base des données provenant du lecteur d'étiquette RFID (42) ; et
un communicateur (58) en communication électrique avec le dispositif de commande (50) pour la communication avec un emplacement distant,
**caractérisé en ce que** le dispositif de commande (50) est configuré pour :
déterminer une gamme de couleurs acceptable pour le produit alimentaire (30) sur la base de l'identification du type du produit alimentaire (30) ; et
déterminer si une couleur actuelle du produit alimentaire (30) mesurée par le capteur de couleur se situe dans la gamme de couleurs acceptable.

2. Ensemble selon la revendication 1, comprenant également au moins un biocapteur en communication électrique avec le dispositif de commande.

3. Ensemble selon la revendication 2, dans lequel l'au moins un biocapteur est configuré pour mesurer des émissions de gaz émises par le produit alimentaire.

4. Ensemble selon la revendication 2, dans lequel l'au moins un biocapteur comporte au moins un capteur chimique ayant un élément biocomposant réactif, un élément capteur et un élément d'interface disposé entre eux.

5. Ensemble selon la revendication 1, comprenant également au moins une cellule solaire (46) en communication électrique avec une batterie (48) pour alimenter l'ensemble (32).

6. Ensemble selon l'une quelconque des revendications 1 à 5, dans lequel l'ensemble de contrôle de la qualité des aliments (32) est situé à adjacent au refroidisseur réfrigéré (20).

7. Ensemble selon la revendication 1, dans lequel le dispositif de commande est également configuré pour régler la température d'un système de réfrigération afin de réduire la détérioration des aliments si le changement de couleur du produit alimentaire dépasse un niveau prédéterminé.

8. Ensemble selon l'une quelconque des revendications 1 à 5, dans lequel l'ensemble de contrôle de la qualité des aliments (32) est situé à l'intérieur d'une remorque de transport d'aliments.

9. Ensemble selon la revendication 1, dans lequel le dispositif de commande comporte un microprocesseur et une mémoire pour stocker des données de produit alimentaire, dans lequel les données de produit alimentaire comportent la gamme de couleurs acceptable pour le produit alimentaire.

10. Procédé de surveillance de l'état d'un produit alimentaire (30) comprenant les étapes suivantes :
l'identification d'un type du produit alimentaire sur la base de données provenant d'un lecteur d'étiquette RFID (42) lisant une étiquette RFID sur le produit alimentaire
la détermination d'une gamme de couleurs acceptable pour le produit alimentaire (30) sur la base de l'identification du type du produit alimentaire (30) ;
la mesure d'une couleur actuelle du produit alimentaire (30) avec au moins un capteur de couleur (36) ;
le fait de déterminer si la couleur actuelle se situe dans la gamme de couleurs acceptable pour le produit alimentaire (30) ; et
le signalement à un utilisateur si la couleur actuelle est en dehors de la gamme de couleurs acceptable pour le produit alimentaire (30).

11. Procédé selon la revendication 10, dans lequel l'étape de signalement à l'utilisateur comporte l'allumage d'une lumière (62) .

12. Procédé selon la revendication 10, comprenant également la mesure d'un niveau d'émissions de gaz associé au produit alimentaire (30) à l'aide d'au moins un biocapteur.

13. Procédé selon la revendication 12, comprenant également un signalement à l'utilisateur si le niveau d'émissions de gaz est en dehors d'une plage d'émissions de gaz prédéterminée.

14. Procédé selon la revendication 10, comprenant également le réglage d'une température d'un système réfrigéré pour réduire la détérioration des aliments si le changement de couleur du produit dépasse un niveau prédéterminé.

15. Procédé selon la revendication 10, dans lequel l'étape de détermination d'une gamme de couleurs acceptable pour le produit alimentaire (30) comporte l'accès à des données de produit alimentaire stockées associées au type du produit alimentaire (30) .
